# EUROPEAN PATENT APPLICATION

(11) **EP 4 484 951 A1**
(43) Date of publication of application: **01.01.2025**
(21) Application number: 23182051.5
(22) Date of filing: 28.06.2023
(51) Int. Cl.: G01N 33/50, G01N 33/564, G01N 33/569, G01N 33/68, G16B 25/10, G16H 50/20

(54) **BAT-BASED METHOD FOR DETERMINING THE PREVALENCE AND/OR SEVERITY OF AN ALLERGY IN AN INDIVIDUAL AND KITS THEREFORE**

(71) Applicant: Forschungszentrum Borstel, Leibniz Lungenzentrum, 23845 Borstel (DE)
(72) Inventor: Walsemann, Theresa, 24113 Kiel (DE); Behrends, Jochen, 22967 Tremsbüttel (DE); Jappe, Uta, 23843 Bad Oldesloe (DE)
(74) Representative: Kröncke, Rolf

(57) **Abstract**

The present invention relates in a first aspect to a method for determining the prevalence and/or severity of an allergy in an individual with or suspected to be afflicted with allergy against an allergy-triggering source whereby the method is a basophil activation test (BAT)-based method substituting the current, potentially risky method of allergy challenge considered as the gold standard of allergy diagnostic measures. In addition, the present invention relates to the method which can be conducted automatically and preferably in a computer-implemented method for fast and reliable determination of the prevalence and/or severity of allergy in said individual. In particular, the present method allows the calculation of a combined symptom and tolerance class (CSTC), which allows the individual to be categorized into different classes of allergy severity, e.g., to guide therapeutic decisions and/or behavioural recommendations for said individual. Moreover, the present invention provides a computer-implemented method for doing so and in a further aspect, a test kit or a kit of parts for determining the prevalence and/or severity of an allergy according to the present invention. The use of the test kit includes the application of a basophil activation test following the method according to the present invention.

## Description

The present invention relates in a first aspect to a method for determining the prevalence and/or severity of an allergy in an individual with or suspected to be afflicted with allergy against an allergy-triggering source whereby the method is a basophil activation test (BAT)-based method substituting the current, potentially risky method of allergy challenge considered as the gold standard of allergy diagnostic measures. In addition, the present invention relates to the method which can be conducted automatically and preferably in a computer-implemented method for fast and reliable determination of the prevalence and/or severity of allergy in said individual. In particular, the present method allows the calculation of a combined symptom and tolerance class (CSTC), which allows the individual to be categorized into different classes of allergy severity, e.g., to guide therapeutic decisions and/or behavioural recommendations for said individual. Moreover, the present invention provides a computer-implemented method for doing so and in a further aspect, a test kit, or a kit of parts for determining the prevalence and/or severity of an allergy according to the present invention. The use of the test kit includes the application of a basophil activation test following the method according to the present invention.

### Prior Art

Prevalence to food allergy varies greatly from region to region but increases steadily, especially in Western industrial nations. Allergic disorders have emerged from a rare disease to one of the most common chronic diseases in Western countries, afflicting approximately 30% of the population. Moreover, the prevalence is still rising and will become a problem for the population of the world. It is predicted that 50% of the European population may be afflicted with allergy in the future.

For example, allergy and allergic reactions to peanut have increased threefold in the US in the past decades, whereby allergy affects about 2% of children in the United Kingdom and the US and being the most common cause of life-threatening anaphylaxis in childhood. Therefore, correct diagnosis and grading is crucial to prevent potentially life-threatening allergic reactions against peanuts, as well as other e.g., food-borne, or environmental allergy-triggering sources (incl. e.g., wasp venom and others). The skin prick-tests (SPT) and immunoglobulin (Ig)E antibody assays have been used to detect sensitization, but do not prove allergy. To detect allergy in unclear cases, challenge tests with suspected allergen sources are currently the diagnostic gold standard. For food allergies, these challenge tests are conducted as oral food challenge (OFC) to determine the prevalence and/or severity of allergy against the allergenic sources. However, challenge tests in general and OFC in particular are very time consuming and associated with inherent risks for the person tested. To be able to provide immediate medical attention in the case of any acute allergic reactions during challenge, the affected person must be constantly monitored, and hence an OFC is often associated with hospitalization. To avoid such dangers and burdens, an attempt is made to establish the diagnosis by thorough medical history and determining sensitivity based on measuring soluble IgE in the blood (serum) and/or via SPT. However, historybased diagnosis combined with measurement of IgE is often not sufficient to allow a clear determination of the prevalence and/or severity of an allergy.

In the last decade, the basophil activation test has been discussed as a method to safely simulate allergic reactions with patient blood *in vitro,* and thus determine the presence and severity of allergy. In this context, several attempts have been made to utilize the BAT to distinguish individuals with asymptomatic sensitization from true allergic individuals and to predict the severity of allergy. However, each of these attempts had its shortcomings. For example, Santos A., et al., J. Allergy Clin. Immunol., 2015, 135 (1), 179 - 186 disclose distinguishing parameters of basophil activation test reflecting the severity and tolerance of allergic reactions to peanut. In addition, Schwager C., et al., report on peanut oleosins associated with severe peanut allergy and the importance of lipophilic allergens for comprehensive allergy diagnosis (see Schwager et al., J. Allergy Clin. Immunol., 2017, Doi: 10.10168/J.Jaci 2017.02.020).

Recently, a new method for the determination of basophil activation by flow cytometry has been described that allows accurate *in vitro* examination of patient samples in high throughput manner. It is thus suitable for routine diagnostics, while maintaining diagnostic performance and overcoming the shortcomings of known prior art methods (see WO 2022/038217 A1).

Diagnosis of peanut allergy is particularly challenging when there is no clear history of peanut consumption, which is often the case in younger children. In fact, due to the increasing awareness of food allergies, parents try to avoid corresponding foods in their children's diet during their first years of life. Regardless, peanut-sensitized children without a history of specific oral peanut exposure make up a significant proportion of patients requiring treatment at allergy clinics, contributing to the increasing number of risk-associated OFCs that have been conducted in recent years.

As a safe alternative, BAT, in which an immediate type I allergy is simulated *in vitro* using patient blood, has been described in the art. Here, aliquots of a blood sample are incubated with different concentrations of a potential allergen or allergy-triggering source. In case of an existing allergy, these stimuli are recognized by specific basophil-coupled IgE's, while in case of sensitization, only soluble (i.e. non-basophil-bound) IgEs are present. The corresponding IgE:allergen complexes bound to basophils lead to the cross-linking of neighboring IgE receptors, eventually resulting in the release of mediators, like histamine, and the expression of specific markers of basophil activation (including CD63 and CD203c) on the surface of the basophils.

As mentioned above, prior art WO 2022/038217 A1 describes a flow-cytometric method that determines the ratio of CD203c-positive and CD63-positive basophils as a measure of allergen-mediated basophil activation. This improved BAT also overcomes shortcomings of conventional BATs, as it can be performed automatically and in high throughput, equally saving costs, time, and labor. Accordingly, this BAT also offers clear advantages over the SPT and/or challenge tests as well as serological assays in terms of safety, sensitivity, and specificity, and also overcomes limitations of conventional BATs *inter alia* regarding the short time window between sampling and analysis (typically less than 6 hours and the high cut off values for positivity of above 15 %), which formerly prevented broader use of the BAT method.

In the literature, different BAT-based methods are described and discussed as a substitute for OFCs and SPT, as well as for the determination of the presence and/or severity of an allergy. Regarding peanut allergy, these previous studies for assessing the allergy status are usually based on a single stimulus, such as a peanut extract (PEX), or on a maximum of two stimuli, such as the serodominant ("major") allergen Ara h 2 combined with the minor allergen Ara h 6. This latter approach was for instance used by van Erp et al. (see van Erp F. C., et. al, 2017, J. Allergy Clin. Immunol.; 139 (1): 358 - 60e8). According to the authors, the detection of slgEs against both allergens allows for a "very good" prediction of the presence of peanut allergy (AUC: 0.95 and 0.93, respectively). Using the best thresholds (highest negative and positive predictive value, PPV and NPV) for Ara h 2, 50 of 82 children (62%) could be correctly classified. Including the results of a BAT (with Ara h 2 and Ara h 6 as stimuli), this rate was increased to 65 of 81 children (80%). The BAT itself also showed good correlation in discriminating allergic from non-allergic children (AUC: 0.84), and is considered by the authors as a complementary, rather than stand-alone tool.

In another report, only PEX was used to discriminate between allergic, sensitized, and non-allergic children. Although the performance of this approach was generally favorable with a 98% sensitivity and a specificity of 96%, the authors observed substantial basophil activation in the presence of PEX in asymptomatic patients, reaching levels of up to 60% degranulated basophils (see Santos A. F., et al., 2014, J. Allergy Clint. Immunol. 134: 645-652). To obtain meaningful results, the authors therefore recommend a two-step process in which a SPT is performed first or (analogous to van Erp et al.) serum is tested for the presence of soluble IgEs (slgEs) against Ara h 2, and the BAT is used only to confirm the diagnosis.

Other studies utilizing PEX as the sole stimulus in BAT have not been able to confirm the results reported by Santos et al., 2014, yielding a sensitivity of only 79% to 81% and specificity of 86% to 95%. Consequently, there is the problem of either false-positive or (if this is to be circumvented by raising the cut-off value) false-positive diagnoses, thus, the results must be confirmed by OFC.

Using the same BAT/PEX-based approach, Santos et al. also attempted to correlate the severity and threshold of an allergic reaction with the presence of slgEs (see Santos et al. (2015) J. Allerg. Clin. Immunol. 135: 179-186 and Santos et al. (2020) J. Allerg. Clin. Immunol. 146: 344-355). Here, the ratio of basophil activation with PEX or anti-IgE (as positive control) served as a marker, which is problematic *per se* because activation with anti-IgE is highly dependent on the antibody used and the method therefore inherently has low reproducibility. Hence, the predictive accuracy was modest (AUC: 0.87), and clinically important differentiation between mild and moderate allergy severity was not possible.

Ocmant et al. (Ocmant et al. (2009) Clin. Exp. Allerg. 39: 1234-1245) and Rentzos et al. (Rentzos et al. (2015) Clin. Tranl. Allerg. 5: 22) also used BATs with PEX to diagnose severe peanut allergy in children (Ocmant et al.) and adults (Rentzos et al.), respectively. Performance was satisfactory at best in both cases (AUC: 0.89 and 0.86, respectively), and in contrast to the study by Santos et al, no correlation with slgE titers against Ara h 2 (and also Ara h 1, Ara h 3, Ara h 6) was observed. Accordingly, both groups of authors refer to their BAT-based methods only as complementary diagnostic tools to, for example, SPT.

Jaumdally et al. (Jaumdally et al. (2022) Pediatr. Allergy Immunol. 33: e13870) investigated the reproducibility of a PEX-based BAT as a surrogate for OFCs and its transferability to the clinical setting. Although the authors concluded positively, the clinicians involved refrained from using an OFC only in the case of a positive BAT and supplemental sensitization data. In the case of a negative BAT, an OFC was still performed in most cases, which turned out to be positive or inconclusive in 50% of all cases. This shows the limitations of an only PEX-based analysis. In addition, the authors found greater variations depending on the type of BAT method used, whereby the improved BAT of WO 2022/038217 A1 was not tested.

That is, the BAT-based methods disclosed in the prior art do not allow (i) to reliably assess the status and/or severity of allergy in individuals, and (ii) to be clinically used as a substitute for risky challenge tests (e.g., OFC). Therefore, there is a need for a robust BAT-based method to determine and classify the allergy status and the severity of allergy in individuals, ideally using only few specific probes or stimuli of the suspected allergy-triggering source.

### Brief description of the present invention

The present inventors aim in providing a BAT-based method that allows reliable assessment of the status and/or severity of allergy in an individual, and which can be used as a dependable substitute for potentially risky challenge tests (e.g., OFC) otherwise required for this purpose.

That is, the present invention relates in a first aspect to a method for determining the prevalence and/or severity of an allergy in an individual afflicted with or suspected to be afflicted with allergy against an allergy-triggering source,
comprising the steps of:
a) providing a sample of the said individual;
b) aliquoting the sample and performing an allergen-resolved BAT (basophil activation assay) wherein a single aliquot is incubated with exactly one stimulus selected from i) to v):
   i) an extract of the allergy-triggering source;
   ii) a major allergen of the allergy-triggering source;
   iii) optionally, a minor allergen of the allergy-triggering source;
   iv) one or more lipid-associated allergen(s) of the allergy-triggering source wherein the one or more lipid-associated allergen(s) is different to the major allergen of ii) and/or the optional minor allergen of iii); and
   v) no allergen of the allergy-triggering source as control;
c) determining the basophil activation for each of the individual aliquots i) to v) from step b),
d) determining the change of CD63 expression and CD203c expression in the individual aliquots i)-iv) compared to the control v); and
e) calculating a combined symptom and tolerance class (CSTC) as a measure for the prevalence and severity of an allergy against the allergy-triggering source for the individual based on the data determined in steps c) and d).

In a further aspect, the present invention relates to a flow cytometric method, wherein the fluorescence-labelled markers anti-CD63, anti-FcεR1a, and anti-CD203c are being used to extract and determine (i) the proportion of CD63- and CD203c-positive basophils as a measure of basophil activation, as well as (ii) the change in median fluorescence intensity (delta-MFI) of CD63 and CD203c (in relation to the control) as a measure of the change of CD63 and CD203c expression.

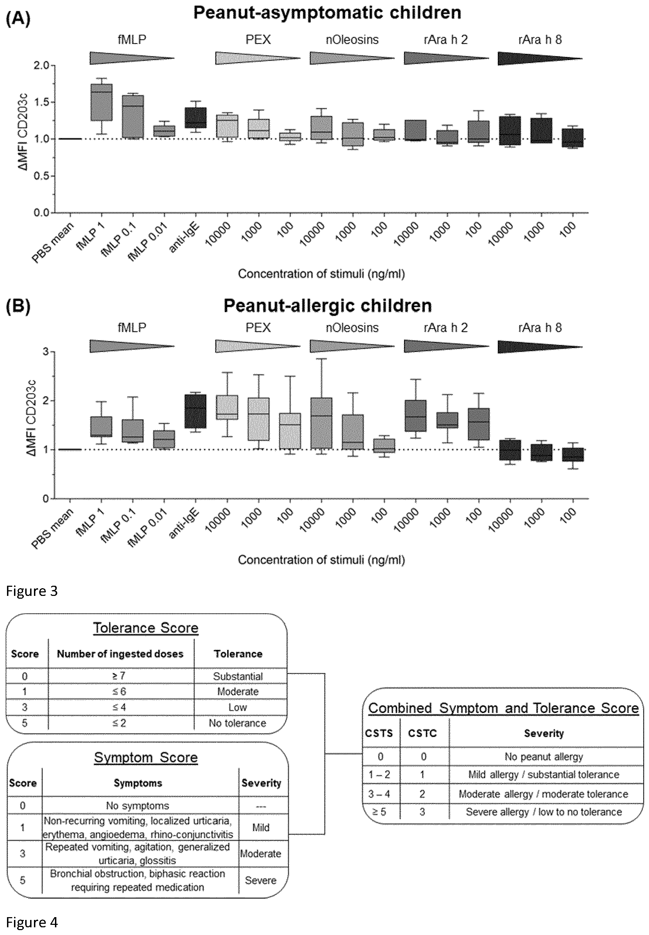

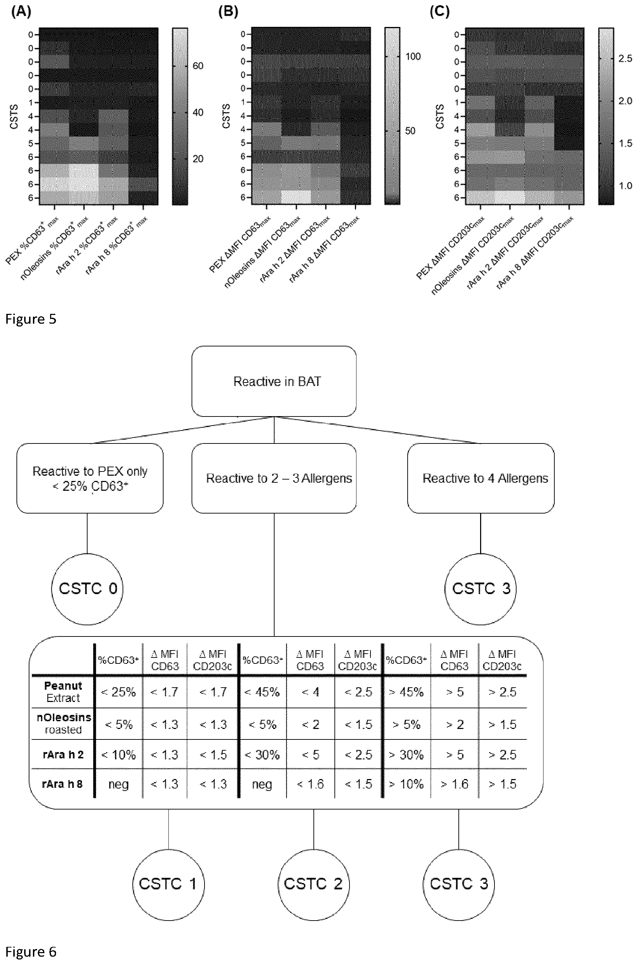

In an embodiment of the present invention, the CSTC is categorized into the stages 0, 1, 2, or 3, whereby these different CSTC stages differentiate between individuals with high tolerance and no allergy (CSTC 0), mild allergy and substantial tolerance (CSTC 1), moderate allergy and moderate tolerance (CSTC 2), and severe allergy and low to no tolerance (CSTC 3). This CSTC categorization into 4 classes as well as the CSTS categorization as described herein are based on the guidelines outlined in Ring J,, Allergo J. 2021;30(1):20-49 and Chinthrajah RS, et al., J Allergy Clin Immunol. 2022;149(6):2166-70 e1. Namely, the CSTS is based on said guidelines as shown in figure 4 and the CSTC is based on the guidelines with the parameters identified in figure 6.

In a further aspect, the present invention relates to a computer-implemented method.

In addition, a computer-readable medium or computer-program product having an executable instruction for performing the method according to the present invention are provided as well as the use of a test kit or kit of parts for determining the prevalence and/or severity of an allergy in an individual afflicted with or suspected to be afflicted with allergy against an allergy-triggering source.

### Brief description of the drawings

Figure 1: Proportion of CD63-positive basophils in BAT
   Figure 1A-B: Activation of basophils in response to stimuli and controls in BAT for peanut sensitized-only patients (N = 4, A) and peanut-allergic patients (N = 8, B). Visualized as box-whisker plot, boxes depict median and IQR, whiskers depict minimum and maximum. PEX = peanut extract
Figure 2: Fold change of CD63 median fluorescence intensity in BAT Figure 2 A-B: Upregulation of CD63 expression on the cell surface of basophils in response to stimuli and positive controls compared to PBS in basophil activation test for peanut sensitized-only patients (N = 4, A) and peanut-allergic patients (N = 8, B). Visualized as box-whisker plot, boxes depict median and IQR, whiskers depict minimum and maximum. PEX = peanut extract
Figure 3: Fold change of CD203c median fluorescence intensity in BAT Figure 3 A-B: Upregulation of CD203c expression on the cell surface of basophils in response to stimuli and positive controls compared to PBS in BAT for peanut sensitized-only patients (N = 4, A) and peanut-allergic patients (N = 8, B). Visualized as box-whisker plot, boxes depict median and IQR, whiskers depict minimum and maximum. PEX= peanut extract
Figure 4: Scoring system for the tolerance threshold and symptoms during OFC CSTS = combined symptom and severity score, CSTC = combined symptom and severity class
Figure 5: Heat map of BAT parameters and combined symptom and tolerance score A: Maximum percentage of activated basophils observed during BAT for each stimulus and study participant B-C: Maximum fold change of CD63 (B) or CD203c (C) MFI compared to PBS observed during BAT for each stimulus and study participant.
   CSTS = combined symptom and tolerance score
Figure 6: Algorithm for BAT analysis to predict peanut allergy and severity
   PEX= peanut extract. MFI = median fluorescence intensity. CSTC = combined symptom and tolerance class.
Figure 7: Performance of BAT for prediction of allergy prevalence and severity
   A: ROC curve for each BAT stimulus and the combined algorithm to differentiate between peanut allergy and asymptomatic sensitization.
   B: Performance evaluation of ROC to predict prevalence of an allergic reaction.
   C: Evaluation of algorithm for predicting the CSTC of study participants
   PEX= peanut extract, CSTC = combined symptom and tolerance class

### Detailed description of the present invention

In the first aspect, the present invention relates to a method for determining the prevalence and/or severity of an allergy in an individual afflicted with or suspected to be afflicted with allergy against an allergy-triggering source,
comprising the steps of:
a) providing a sample of the said individual;
b) aliquoting the sample and performing an allergen-resolved BAT (basophil activation assay) wherein a single aliquot is incubated with exactly one stimulus selected from i) to v):
   i) an extract of the allergy-triggering source; or
   ii) a major allergen of the allergy-triggering source; or
   iii) optionally, a minor allergen of the allergy-triggering source; or
   iv) one or more lipid-associated allergen(s) of the allergy-triggering source wherein the one or more lipid-associated allergen(s) is different to the major allergen of ii) and/or the optional minor allergen of iii); or
   v) no allergen of the allergy-triggering source as control;
c) determining the basophil activation for each of the individual aliquots i) to v) from step b),
d) determining the change of CD63 expression and CD203c expression in the individual aliquots i)-iv) compared to the control v); and
calculating a combined symptom and tolerance class (CSTC) as a measure for the prevalence and severity of an allergy against the allergy-triggering source for the individual based on the data determined in steps c) and d).

That is, the present inventors recognized that based on a BAT method it is possible to calculate a combined symptom and tolerant class (CSTC) as a measure or surrogate marker for the prevalence and severity of an allergy against the allergy-triggering source in an individual.

As used herein, the term "prevalence of an allergy in an individual" refers to a Type-I hypersensitivity reaction according to the Gell and Coombs classification, mediated by immunoglobulin E (IgE), and is fast in occurring (less than 30-60 min).

As used herein, the term "the severity of an allergy in an individual" refers to severity degrees reflected by variability in allergen-triggered clinical manifestations and eliciting doses.

The term "combined symptom and tolerance class (CSTC)" as used herein refers to a classification scheme outlining the stage of allergy severity and sensitivity. The CSTC is based on grading the severity of objective symptoms and eliciting dose during an OFC as shown in figure 4.

The term "allergy triggering source" which is used herein interchangeably, refers to several groups of allergen materials such as pollens, mites, food substances, chemicals.

The term "allergen" as used herein refers to a substance, usually a protein, capable of generating an allergic reaction by comprising at least one B-cell epitope.

The term "major allergen" refers to an allergen against which at least 50% of individuals suffering from an allergy have allergen-specific IgE antibodies.

The term "minor allergen" refers to an allergen against which less than 50% of individuals suffering from an allergy have allergen-specific IgE antibodies.

The term " lipid-associated allergen" refers to major or minor allergen(s) able to interact with lipids *via* different structural features such as hydrophobic cavities or specialized domains. According to the present invention, the lipid-associated allergen is different to the major allergen and/or the optional minor allergen used as stimulus ii.) or iii) in the BAT tests described herein.

The method according to the present invention requires at least the incubation of a single aliquot of the blood sample of the individual with a single stimulus as identified above. Namely, the single stimulus is selected from the mentioned stimuli of i) to v) whereby i) is an extract of the allergy-triggering source, ii) is a major allergen of the allergen-triggering source, iii) is one or more lipid-associated allergens of the allergy-triggering source which is different to the major allergen or minor allergen of ii) or iv) and v) represents the control without any allergy-triggering source. Optionally, a further aliquot is incubated with iv) at least one minor allergen of the allergy-triggering source.

Generally, allergy-triggering sources are composed of a multitude of allergenic and non-allergenic compounds. Allergenic compounds present in the allergy-triggering source can be differentiated into major allergen(s) and minor allergen(s). Typically, allergy-triggering sources comprise at least one major allergen, for example, at least two, at least three or more major allergens. In addition, allergy-triggering sources typically comprise at least one minor allergen, for example at least two, at least three or more minor allergens.

In addition to the differentiation into major and minor allergens, the components of an allergy-triggering source can also be subdivided on the basis of their chemical properties, e.g., into water-soluble and lipid-associated allergens, see above. That is, the lipid-associated allergen for use as a stimulus according to the present invention may be a major or minor allergen. According to the present invention, the lipid-associated allergen used as stimuli iv) is different to the major ii) and/or minor iii) allergen to be used as different stimuli in the BAT test. An allergy-triggering source usually also comprises at least one lipid-associated allergen, for example, at least two, at least three or more lipid-associated allergens.

In case of peanuts, these lipid-associated allergens are (or comprise) the oleosins Ara h 10 and Ara h 11, as well as Ara h 14 and Ara h 15 (see e.g. Schwager C, et. al Plos One 2015 Apr 10;10(4):e0123419. doi: 10.1371/journal.pone.0123419. eCollection 2015).

The control (stimulus v)) represents a control, where the control does not contain any additional component, or does not contain any components that lead to activation of the basophils of the individual. In an embodiment, the control may be additionally an IgE-dependent control and/or an IgE-independent control, both representing positive controls. Namely, the IgE-dependent control is based on the use of anti-IgE antibodies, like a mixture of two anti-lgE-antibodies. Further, the IgE-independent control may be fMLP (N-formylmethionine-leucyl-phenylalanine). The use of the mentioned positive controls is particularly useful in identifying non-responder as well as false-negative results.

The basophil activation is determined by known means. It is preferred that basophil activation is determined as described e.g., in WO 2022/038217 A1.

In an embodiment of the present invention, the method according to the present invention is a method wherein the basophil activation is determined as proportion of activated basophils using the identification markers CD63, FcεR1a, and CD203c along with the following gating strategy:
i) expression of FcεR1a and CD203c;
ii) forward scatter area (FSC-A) vs. sideward scatter area (SSC-A);
iii) sideward scatter width (SSC-W) vs. CD203c;
and the proportion of activated basophils is calculated based on CD63-, FcεR1a- and CD203c-positive cells.

That is, the fraction of basophils expressing both CD203c and CD63 represents the activated basophils, while the fraction expressing only CD203c but not CD63 represents the non-activated basophils.

As used herein the term "double positive for" refers to basophils or other cells where the respective molecules are expressed and detected by respective antibodies against said molecules. E.g. a cell, like a basophil, is referred to as double positive for CD203c and FcεRIα, if the cell is expressing both molecules, and (in addition) the corresponding molecules have been recognized/bound by fluorescent-labelled antibodies, so that the resulting fluorescence is above a cut-off value or exceeds a given threshold.

The terms "fluorophore" and "fluorochrome" are used herein interchangeably and synonymously unless otherwise indicated.

The sample of said subject or individual is obtained in advance. Typically, the sample is selected from fluid or enzymatically digested tissue for serial cell suspension derived from said subject. In an embodiment the sample is collected from sputum, blood, including whole blood, saliva, nasal secretion, and urine, preferably the sample is whole blood. In particular, the sample is whole blood comprising anticoagulation compounds. The whole blood may be whole blood obtained from the subject or pre-treated whole blood like purified whole blood. As noted, the whole blood may contain anticoagulation compounds. Typical anticoagulation compounds include EDTA, citrate, and heparin. In an embodiment, the sample is an *in vitro* sample.

Moreover, in an embodiment, the whole blood is treated in advance for lysis of erythrocytes present in said sample when obtained from the subject accordingly.

The sample is aliquoted, and each aliquot is individually treated or stimulated with the single stimulus. Namely, the individual aliquots are treated and potentially stimulated with the stimuli i) to v), respectively, and for each aliquot the amount of basophil activation is determined as described above. Additional stimuli which may be used in the method according to the present invention as single stimulus in further aliquots for BAT testing may comprise further at least a second major allergen or further major allergens, a second or further minor allergens and/or a second or further lipid-associated allergens.

"Treating or stimulating the sample" means that cells present in said sample, including basophils, are brought into contact with the corresponding stimulus.

The allergy-triggering source may be a mixture of proteins or a protein extract from a biological source. Extracts from biological allergen sources inducing allergy include the group of insect venoms, foods, including fruits, vegetables, seeds, legumes, nuts, spices, fish, shellfish, mollusks, foal, meat, and milk. In addition, tree pollen, grass pollen, weed pollen, epidermal and animal proteins, dust and storage mites, insects, parasites, microorganisms, and house dust may be mentioned as sources for the allergy-triggering source.

The stimulus of an allergy-triggering source is added in suitable amounts to the sample. Suitable amounts are known to the skilled person, and typically, the stimulus is added in an amount of 0.1 ng/ml to 10 mg/ml.

To analyze an aliquot that either has been pre-treated with a stimulus or not, a mixture of antibodies is added. Each of these antibodies is labelled with a different fluorochrome. The mixture of antibodies contains labelled antibodies, namely, anti-CD63, anti-CD203c and anti-FcεRIα antibodies.

The antibodies may be commercially available antibodies of different species. Typically, the antibodies are monoclonal antibodies labelled with a fluorochrome. However, it is also possible, that the antibodies are not directly labelled, but rather recognized by a labelled secondary antibody directed against the primary antibody, namely, any one of the antibodies of anti-CD63, anti-CD203c and anti-FcεRIα.

The cells present in a treated or stimulated aliquot are incubated with said mixture of antibodies. Typically, after sufficient incubation time to allow binding of the antibodies to the specific target molecules, the cells contained in the aliquot are washed with a suitable washing buffer. Suitable washing buffers are preferably selected from any kind of washing buffers known in the prior art to neutralize the action of the lysis reagent and to remove interfering factors and cell debris after erythrocyte lysis.

After incubation with the labelled antibodies, the aliquot is measured by flow cytometry. Suitable devices that enable flow cytometric measurements are known to the skilled person. Devices for flow cytometric measurements contain, among other things, suitable light sources, typically lasers, for excitation of the fluorescent dyes attached to the antibodies. Moreover, suitable detectors for measuring the different parameters are present. Typically, the parameters measured by flow cytometry include at least the following: forward scatter signal, including the height and area thereof, a side scatter signal, including the area and height thereof, and the fluorescence of the labelled antibodies. The present detectors allow the differentiation and specific measurement of the fluorescence emitted by the different fluorochromes. The detectors include suitable optical devices for specific measurement of the different fluorochromes.

The data obtained from the detectors are further processed. Namely, basophil activation in aliquots based on data obtained from flow cytometric measurement. The corresponding analysis comprises several steps. Typically, the first step is the exclusion of doublets of cells, which can be achieved by analyzing the data of the forward scatter area vs. forward scatter height. Only single cells are gated and further analyzed in the next step for the expression of the basophil identification markers FcεRIα and CD203c. Single cells that are double positive for these two antigens, namely FcεRIα and CD203c, are gated as basophil-containing fraction.

The basophil-containing fraction is then further analyzed based on the forward scatter area vs. the side scatter area, to differentiate the constituting single cells according to size and granularity. Since basophils are granulated cells, this step allows to exclude nongranulated cells based on the side scatter area.

The fraction of granulated cells obtained from the step above is then analyzed for forward-scatter width and the basophil activation marker CD203c, and the activation status of the basophils is determined based on the double-positivity for both activation marker CD203c and CD63. Cells expressing both, CD203c and CD63, are regarded as activated basophils and, consequently, it is possible to determine the percentage of basophil activation, i.e., the proportion of activated basophils compared to non-double positive cells (CD203c positive / CD63 negative).

In addition to the determination of basophil activation, the change of CD63 and CD203c expression can be determined based on the change of the median fluorescence intensity (MDI) of both markers, when compared to the non-stimulated control v) with the data obtained.

In an embodiment, a further step is included wherein the basophils obtained in step iii), namely by gating on forward scatter area vs. side scatter area, are further analyzed on the side scatter area vs. the measured fluorescence of the antibody against FcεRIα. FcεRIα-positive basophils are gated and further analyzed for the expression of CD203c vs. expression of CD63. Preferably, the basophil activation is determined using fluorescence labeled markers of anti-CD63, anti-FcεRIa and anti-CD203.

Beside the determination of the basophil activation, the change of CD63 expression as well as CD203c expression is determined in each of the aliquots tested.

Typically, the expression of the mentioned markers (CD63 and CD203c) is measured as their expression on the surface of the basophils. For example, the change of the expression of CD63 and CD203c is determined as the change of median fluorescence intensity in cytometric measurement, which typically expressed as x times or x fold the control. The skilled person is well aware of the necessary requirements for determining the change of median fluorescence intensity in cytometric measurement. Of course, other quantitative or semi-quantitative measurements are also possible that allow determination of the change in expression.

Based on said parameters, namely the basophil activation in each of the individual samples as well as the change in expression of CD63 and CD203c, a combined symptom and tolerance class (CSTC) is calculated. The calculation incorporates the multifactorial aspects of the different parameters that allow for classification into different stages or degrees of allergy (severity of allergy) as well as the degree of tolerance to the allergy-triggering source. The CSTC thus represents for the individual a classifier or measure for the prevalence and severity of an allergy against the allergy-triggering source based on the data determined.

In an aspect, the method allows to differentiate between allergic individuals and sensitive but non-allergic individuals. Namely, it is possible to identify those individuals who are sensitized but tolerant to the allergy triggering source.

In case of peanut allergy, the extract is an extract from peanut, like an alkaline peanut extract obtained by methods known in the art. For peanut, major allergens described in the art include Ara h 2, Ara h 1, Ara h 3.

As minor allergens Ara h 8; Ara h 5; Ara h 6; Ara h 7; Ara h 9; Ara h 10; Ara h 11; Ara h 12; Ara h 13; Ara h 14; Ara h 15; Ara h 16; Ara h 17; and Ara h 18 are described. The lipid-associated allergens described for peanut are oleosins Ara h 14 and Ara h 15 from roasted peanuts as well as Ara h 10 and Ara h 11. In https://doi.org/10.3389%2Ffimmu.2019.00122, Jappe U., et al., describe lipophilic allergens or lipid-associated allergens, respectively.

In an aspect, the present invention relates to a method according to the present invention comprising further the step of excluding individuals not applicable to BAT, namely, individuals which are characterized by non-responsiveness to IgE-mediated basophil activation. To allow exclusion of said individuals, the positive controls mentioned above are included in the BAT, i.e., the IgE-dependent stimulus and, optionally, the IgE-independent stimulus to exclude false-negative results.

It is envisaged that the method according to the present invention can be conducted, at least in part, by a computer-implemented analysis method.

In one aspect, the method according to the invention allows classification into different stages of CSTC, whereas the corresponding classification is based on predetermined thresholds for the basophil activation and the change in the expression of CD63 and/or CD203c determined for the different stimuli.

As further outlined in the examples and the figures, CSTC can be divided into the stages of CSTC 0, CSTC 1, CSTC 2, and CSTC 3, as a surrogate marker of the degree of allergy and tolerance to the allergen-triggering source (see e.g. table 1).

A CSTC 0 stage is characterized by reactivity only to extract i) with a proportion of activated basophils of less than 25%. CSTC 0 indicates the absence of allergy and a high tolerance to the allergy-triggering source.

A CSTC 1 is typically characterized by reactivity to no more than three stimuli i) to iv), where the proportion of activated basophils for the extract i) is less than 25%, the proportion of activated basophils for each of the other stimuli ii) to iv) does not exceed 10%, and the change in expression of CD63 and CD203c, e.g. expressed as delta MFI, is less than 2-fold. CSTC 1 indicates mild allergy and a substantial tolerance to the allergy-triggering source.

Further, CSTC 2 is typically characterized by reactivity to not more than three stimuli i) to iv), where the proportion of activated basophils only for one stimulus exceeds 30% and the change in expression of CD63 and CD203c for most stimuli is less than 2.5 fold. CSTC 2 indicates a moderate allergy and moderate tolerance to the allergy-triggering source.

Finally, CSTC 3 is typically characterized by reactivity to at least three stimuli i) to iv), where the proportion of activated basophils for at least two stimuli exceeds 30%, and the change of expression of CD63 expression and CD203c for most stimuli is above 2.5 fold. CSTC 3 indicates a severe allergy and a low or no tolerance to the allergy-triggering source.

In an embodiment, the method according to the present invention is a method where the CSTC is used to guide behavioral recommendations and/or therapy decision for the individual based on the CSTC determined.

In addition, the present invention provides a computer-implemented method for determining the prevalence and/or severity of an allergy of an individual suspected to be afflicted with allergy against an allergy-triggering source comprising the step of
a) obtaining data of measured parameters, preferably by flowcytometry, to determine the percentage or absolute number of activated basophils at least in individual samples i) to v) as defined above of the individual, wherein the basophil activation is determined using the identification markers CD63, FcεR1a, and CD203;
b) computing the data of step a);
c) identifying the percentage or absolute number of activated CD63 expressing basophils, the change of CD63 expression, and the change of CD203c expression of said activated basophils compared to the control v) as defined above;
d) determining the allergy status and/or severity, optionally showing the data on an output unit.

The present invention relates further to a computer medium or computer program product having computer executable instructions for performing the steps as identified in a method according to the present invention.

Moreover, a test kit or a kit of parts is provided. Said test kit or kit of parts is for determining the prevalence and/or severity of an allergy in an individual afflicted with or suspected to be afflicted with allergy against an allergy-triggering component comprising anti-CD-203c, anti-FcεRIα and anti-CD63 antibodies, each labelled with a distinct fluorochrome, optionally lysis buffer for erythrocytes and, optionally, washing buffer, and, in addition, instruction on how to use the test or kit of parts in a method according to the present invention. The test kit or kit of parts may further comprise lysis buffer for erythrocytes and, optionally, washing buffer. Moreover, the test kit or kit of parts may contain one or more test substances used for activating the basophils.

The test kit or the kit of parts according to the present invention is useful for determining basophil activation by flow cytometric measurement. In an embodiment, basophil activation is induced by a test substance *in vitro.*

In a further embodiment, the test kit or kit of parts further comprising at least the extract of the allergen triggering component, the major allergen, and the lipid-associated allergen(s).

In addition, the method according to the present invention is a method wherein the sample processing and measurement are conducted automatically using a flow cytometer with integrated robotic sample handling and manipulation. In a further embodiment, the basophil activation is determined using the computer-implemented method according to the present invention.

The present invention will be described further by way of examples without limiting the invention thereto.

### Stimulation of whole blood basophils

Heparinized blood (Vacutainer LH 170, BD, Heidelberg, DE / S-Monovette^{®} 9 ml LH, Sarstedt) from study patients was obtained and stored at 4°C after blood sampling. The samples were further processed within 48 h. The basic method has been developed in a cooperation of the research group Clinical and Molecular Allergology with the Core Unit Fluorescence Cytometry of the Research Center Borstel [Behrends J, et al., Allergy. 2021;76(12):3776-88]. It is based on the ex *vivo* stimulation of vital basophil granulocytes in whole blood with different stimuli and subsequent following flow cytometric analysis. All used stimuli are listed in Table 1 below.

For the further evaluation of the BAT as a replacement for allergen challenges, peanut allergy was used as a model disease due to sample availability and the possibility to test patients that were simultaneously challenged in a controlled setting provided by an OFC. A comprehensive panel of peanut allergens was chosen for analysis (refer to Table 1). Alkaline PEX and dialyzed oleosins isolated from roasted peanuts, were produced as described e.g. in Schwager C, et.al., PLoS One. 2015;10(4):e0123419. The recombinant peanut allergens Ara h 2 and Ara h 8 were purchased commercially (Indoor Biotechnologies, Cardiff, UK).

Stock solutions of all stimuli were prepared with PBS at a concentration 10x higher than the final concentration indicated in Table 1. fMLP and allergens were then serially diluted 1:10 so that three concentrations per stimulus were obtained. 10 µl of each stimulus at each concentration was pipetted into a round-bottom 96-well microtiter plate, topped by 90 µl of the heparinized blood that was gently agitated beforehand, and mixed by carefully pipetting up and down. The plate was incubated in a closed stainless-steel box on a wetted paper towel at 37°C for 30 min.

**Table 1: Controls and allergens used as stimuli in basophil activation test.**

| **Stimulus** | **Final Concentration** | **Function** |
|---|---|---|
| PBS | --- | Negative control |
| fMLP (Formyl-Methionyl-Leucyl-Phenylalanine) | 1 µM, 0.1 µM, 0.01 µM | Positive control (IgE-independent) |
| Anti-IgE A | 1 µg/ml | Positive control (IgE-dependent) |
| Anti-IgE B | 1 µg/ml | Positive control (IgE-dependent) |
| Anti-IgE A + B | 1 µg/ml | Positive control (IgE-dependent) |

| **BAT design for peanut allergy** | | |
|---|---|---|
| Alkaline peanut extract (PEX) | 10 µg/ml, 1 µg/ml, 0.1 µg/ml | Allergen mix |
| Oleosins from roasted peanuts | 10 µg/ml, 1 µg/ml, 0.1 µg/ml | Allergen mix |
| Recombinant Ara h 2 | 10 µg/ml, 1 µg/ml, 0.1 µg/ml | Single allergen |
| Recombinant Ara h 8 | 10 µg/ml, 1 µg/ml, 0.1 µg/ml | Single allergen |

After stimulation, the blood was stained in a darkened room with 35 µl each of a mix of fluorophore-conjugated antibodies as listed in Table 2. The plate was incubated for 40 min at 4°C under light protection. The red blood cells were subsequently lysed by adding 200 µl 1-Step Fix/Lyse Solution (eBioscience Inc., San Diego, USA) and incubated for 10 min protected from light at RT. Following a centrifugation step at 200 x g for 5 min at 4°C, the supernatant was decanted by a swinging motion, and the lysis step was repeated. After washing two times with 200 µl of PBS each, the pellet was resuspended in 280 µl of MacsQuant Running Buffer (Miltenyi Biotec, Hamburg, DE). The samples were stored at 4°C and analyzed by flow cytometry within 24 h.

**Table 2: Composition of the staining antibody mix used for basophil activation test.**

| **Antibody** | **Conjugate** | **Dilution** | **Manufacturer** |
|---|---|---|---|
| Anti-Human CD63 (clone H5C6) | APC | 1:16 | BioLegend |
| Anti-Human CD203c (clone NP4D6) | PE | 1:8 | BioLegend |
| Anti-Human FcεRIα (clone AER-37) | PE/Cy7 | 1:16 | BioLegend |

### Flow cytometry

Basophils contain intracellular histamine granules with the CD63 antigen associated to the vesicle membrane. If histamine release occurs, the antigen is relocated to the cell surface and can be measured as an activation marker via flow cytometry. After stimulation, the lysed and fixed samples were measured on a LSR II or Symphony A1 flow cytometer (BD Biosciences, Heidelberg, DE) with an adjacent HTS loader (high throughput sampler). The sample processing settings are listed in **Table 3.** Signals were recorded via FACSDiva Software and the gating strategy identified basophils as FcεRIα⁺/ CD203c⁺ cells after doublet exclusion. Activation was defined as CD63⁺ within the gated CD203c⁺ cells and the proportion of activated basophils was calculated percentage of CD63⁺ cells (%CD63⁺). The recorded data were analyzed using FCS Express 7, and exported data were processed with GraphPad Prism.

**Table 3: HTS loader settings for flow cytometry**

| Throughput mode | Standard |
|---|---|
| Flow | 2.5 µl/sec |
| Sample volume | 250 µl |
| Mixing volume | 100 µl |
| Mixing speed | 250 µl/sec |
| Mixes | 5 |
| Wash volume | 800 µl |

### Results: Replacement of allergen challenges by BAT

The interaction of peanut allergens with patients' primary cells, namely whole blood basophil granulocyes, have been investigated using the BAT for improving the diagnosis of allergic patients in clinical routine. As this method is used by few experienced centers as an additional allergy *in vitro* diagnostic test for indirect IgE-detection, the aim of the present invention was to evaluate not just simply the IgE reactivity, but moreover the potential of the BAT to replace *in vivo* allergen challenges, which can be both, time-consuming and unpleasant for patients as they may experience severe allergic reactions during such an allergen challenge. Therefore, a component-resolved BAT containing single allergens identified as important in the allergic disease, has been studied as to whether it makes these challenges unnecessary to diagnose an allergy in the future as well as to give suggestions or recommendations for immunotherapy as a preventative measure.

For the further investigation of the BAT's potential to replace allergen challenges, peanut allergy was chosen as a model disease because the controlled and well-monitored setting provided by an OFC is suited best for a proof-of-principle study in this matter. In addition, symptoms experienced in food allergy, especially mild ones, are often better objectifiable than those of a rhino-conjunctival or bronchial allergy.

### Development of an analysis algorithm to replace allergen challenges: Component-resolved BAT in peanut allergy

### Study cohort

In total, 15 children with a suspected peanut allergy and a positive test for slgE against at least one peanut allergen that were scheduled for an OFC were recruited. Heparinized blood for the BAT was acquired before the start of the OFC. The challenge was carried out single-blinded, and partially defatted peanut flour was administered in halflogarithmically increasing doses with a total of 7 doses. The symptoms developed during the challenge were documented in detail, and the challenge was terminated as soon as objective symptoms occurred. Children were classified as peanut-allergic or asymptomatic (peanut-sensitized but non-allergic) based on their challenge results, wherein non-allergic was defined as successful ingestion of 9.8 g of partially defatted peanut flour on two subsequent days each without symptoms (negative food challenge). The clinical characteristics of the study groups are listed in table 4.

**Table 4: Characteristics of children with suspected peanut allergy.**

| **Patients** | | **Peanut-allergic** | **asymptomatic** |
|---|---|---|---|
| | | **n = 9** | **n = 6** |
| **Age, mean (range)** | | 8 (1-17) | 2 (1-4) |
| **Sex (m/f)** | | 7/2 | 5/1 |
| **Total IgE, mean (95%CI)** | | 273.4 (±254.2) | 548.9 (± 557.7) |
| **Specific IgE concentrations** | | | |
| | Peanut extract, mean (95%CI) | 37.0 (± 28.4) | 1.29 (± 0.48) |
| | rAra h 2, mean (95%CI) | 24.7 (± 19.7) | 0.35 (± 0) |
| **Allergic diseases** | | | |
| | Allergic asthma, N (%) | 1 (11 %) | 0 (0%) |
| | Atopic eczema, N (%) | 5 (56%) | 6 (100%) |

### Cross-evaluation of BAT and OFC results

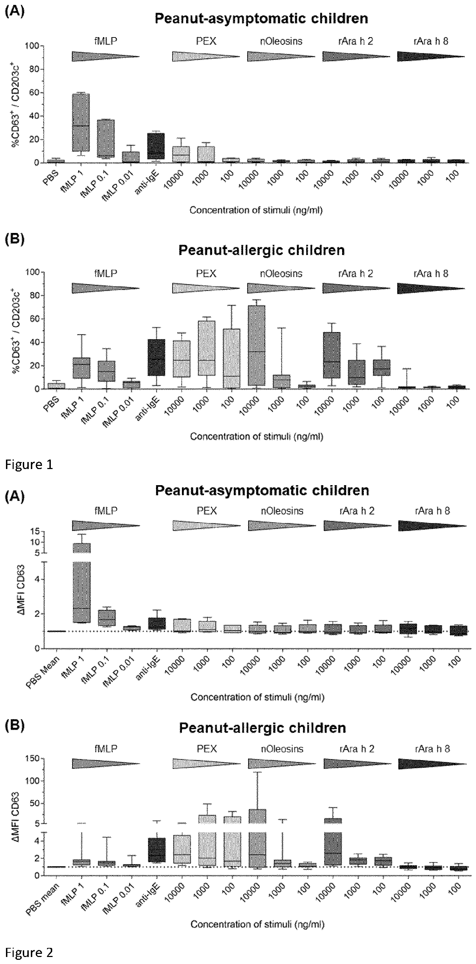

The results of the BAT were compared to the OFC outcomes for each patient, and the children were grouped according to their allergy status. 13% of the participants were nonresponders in the BAT, which is indicated by non-responsiveness to anti-IgE positive controls or any allergen stimuli but normal response to fMLP, which leads to a non-IgE-mediated degranulation by G-protein coupled receptor (GPCR) signaling. All the following analyses only entail the responsive patients of which 5 exhibited an asymptomatic peanut sensitization, and 8 were diagnosed with peanut allergy according to the OFC results. The parameters monitored in the flow cytometric analysis of the BAT after isolating the basophils entailed the percentage of CD63-positive cells in the gated basophils (%CD63⁺, Figure 1), which corresponds to the proportion of degranulated basophils, as well as fold change of median fluorescence intensity (ΔMFI or delta MFI) compared to the negative control PBS for CD63 and CD203c, see Figure 2 and Figure 3, respectively.

While in theory, degranulation of the basophils should only occur if the tested individual is truly allergic, as it mimics the allergic reaction ex *vivo,* 60% of the study participants with negative food challenge results reacted to PEX with substantial degranulation and upregulation of CD63 and CD203c expression as demonstrated in Figure 1A, Figure 2A, and Figure 3A. However, none of the other allergens led to activation of the basophils in sensitized-only study participants.

In addition to PEX, patients with positive outcome of the OFC often reacted to recombinant Ara h 2 and naturally purified, roasted oleosins, the latter of which caused the highest proportion of CD63-positive cells with up to 80% degranulated basophils, Figure 1B. Recombinant Ara h 8, which is described in the literature as a marker for cross-reactivity with the birch pollen allergen Bet v 1 and thus indicates pollen-associated peanut allergy, only led to basophil activation in three patients and only one of those showed a substantial response of 17% CD63⁺ basophils, whereas the others did not exceed 4% of activated basophils.

The fold change of CD203c expression on the basophil surface was higher in patients with peanut allergy than in merely sensitized patients, but overall, the observed changes were moderate with a less than 3-fold increase as the maximum response (Figure 3), whereas the expression of CD63 increased up to over 100-fold in some patients, even though these extreme cases were rather rare (Figure 2). Notably, this hyperresponsiveness occurred only in response to allergens and not the anti-IgE positive control, and not all the allergens that led to a basophil degranulation did necessarily induce a hyperresponsive CD63 ΔMFI in the same patient. However, the ΔMFI of CD63 was generally more responsive when the basophils were activated by stimulation with allergens, which was expected as it is, in contrast to CD203c, not constitutively present on the cell surface, and its upregulation therefore depends on degranulation of the basophils. Overall, all three of the monitored parameters showed substantial differences between allergic and sensitized patients, which confirmed their potential for differentiating true allergy from a clinically silent sensitization.

### Development of a scoring system and BAT analysis algorithm for prediction of allergy prevalence and severity

Since regular scores to assess the severity of food allergy usually only entail the experienced symptoms, a scoring system that included both the tolerance threshold and symptoms occurring during the OFC was developed to classify the patients according to their OFC results and to provide a basis for decision-making with regards to the BAT, becoming a reliable substitute for OFCs. Separate scores for tolerance and symptom severity, only including objective symptoms as these are pivotal for terminating the OFC, were integrated into a combined symptom and tolerance score (CSTS), wherein the symptom scale was based on the CoFAR Grading Scale for Systemic Allergic Reactions and the official German guidelines for the management of anaphylaxis (Ring J, et al., Allergo J. 2021;30(1):20-49). This approach followed in principle the combined symptom and medication scores often used to assess the success of immunotherapy of inhalant allergies. Patients were further classified into combined symptom and tolerance classes (CSTC) according to their scores, wherein CSTC 0 corresponded to a negative food challenge result, and CSTC 1-3 to positive food challenge results with increasing symptom severity and/or decreasing tolerance threshold. The detailed scoring system is summarized in Figure 4. A single score was used to assess symptom severity, which corresponded to the score of the most severe symptom experienced during the OFC. While CSTC 1 was the threshold of a diagnosed allergic reaction, it was associated with mild symptoms and a substantial peanut tolerance, e.g., corresponding to the amount of peanut in a snickers bar. These patients were advised to maintain small amounts of peanut in their diet to sustain the tolerance, whereas strict peanut abstinence was recommended for patients with CSTC 2-3.

A visualization of the individual CSTS values of participants and their corresponding BAT parameters revealed that all the monitored parameters (%CD63⁺, ΔMFI CD63 and ΔMFI CD203c) increased with the scores and therefore confirmed the relation of the BAT results to both tolerance threshold and symptom severity, Figure 5). In particular, the proportion of CD63-positive basophils and the respective fold change in MFI was often massively increased in patients with the highest scores. In contrast, utilizing only either the symptom or the tolerance score was less suitable, especially for patients with high tolerance but severe symptoms and vice versa during the OFC. Overall, the results attested each parameter a promising correlation to the CSTS which is based to the symptoms, that could be used to classify the patients according to their BAT outcomes.

By matching the corresponding CSTC stages of the children with the proportion of activated basophils and fold- change of the MFI of CD63 and CD203c, thresholds of all three parameters were established that corresponded best to the observed outcomes of the food challenge. An analysis algorithm for all patients that were reactive to at least one of the stimuli in BAT was developed to differentiate between the different CSTCs by considering the number of stimuli the patients reacted to as well as the individual proportion of activated basophils and increases in MFI of CD63 and CD203c. The algorithm is outlined in detail in Figure 6.

The thresholds were only applied to stimuli (allergens) the individual reacted to, wherein a positive reaction was defined as a signal exceeding the negative control by 2.5 times and at least 1.5% of degranulated basophils in total. Therefore, even if a single threshold was exceeded, such as "< 5% CD63⁺ cells" when stimulated with natural oleosins, the value was not taken into account for the algorithm if it did not fulfill the criteria for a positive reaction. As soon as one of the parameters' values to differentiate CSTC 1-3 was exceeded, the patients were assigned to the next-higher level until the criteria were met. For the placement in CSTC 3, at least one of the criteria needed to match.

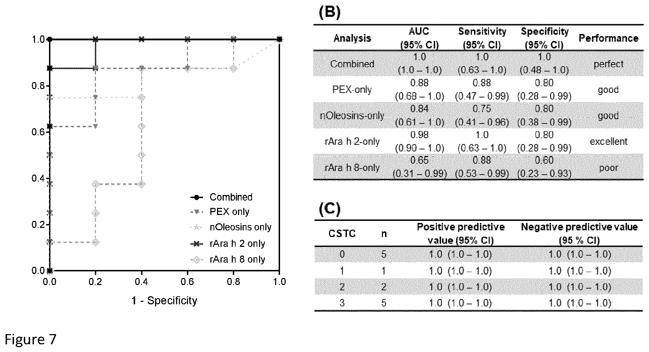

For performance evaluation regarding the ability to differentiate between silent sensitization and actual peanut allergy, each allergen and the combined algorithm were analyzed using a receiver-operator-characteristics (ROC) curve and rated after the respective area under the curve (AUC), see Figure 7A-B, according to the criteria established by Luna-Herrera J, et al. Eur J Clin Microbiol Infect Dis. 2003;22(1):21-7.9. Overall, rAra h 2 performed best as a single parameter to predict the prevalence of peanut allergy. While oleosins were highly specific for severe cases of allergy, the lack of reactions by patients with mild and moderate symptoms towards them, as can be seen in Figure 5, rendered them unsuitable for the differentiation between silent sensitization and allergy. Overall, none of the single parameters reached the sensitivity and specificity of the analysis algorithm in terms of diagnosing the prevalence of a clinically manifest allergic reaction. Furthermore, the developed algorithm was able to reliably differentiate between the different severity grades established by the CSTC system, see Figure 7C. All patients that were responsive in BAT were classified correctly by the analysis algorithm, leading to a positive and negative predictive value of 100% for all responders, which corresponded to 87% of the total study population. Overall, the herein newly described CSTC system combined with the BAT analysis algorithm proved highly effective in the prediction of allergy prevalence and severity of all patients with the only drawback of a non-responder rate of 13%.

## Claims

1. Method for determining the prevalence and/or severity of an allergy in an individual afflicted with or suspected to be afflicted with allergy against an allergy-triggering source,
comprising the steps of:
a) providing a sample of the said individual;
b) aliquoting the sample and performing an allergen-resolved BAT (basophil activation assay) wherein a single aliquot is incubated with exactly one stimulus selected from i) to v):
i) an extract of the allergy-triggering source;
ii) a major allergen of the allergy-triggering source;
iii) optionally, a minor allergen of the allergy-triggering source;
iv) one or more lipid-associated allergen(s) of the allergy-triggering source wherein the one or more lipid-associated allergen(s) is different to the major allergen of ii) and/or the optional minor allergen of iii); and
v) no allergen of the allergy-triggering source as control;
c) determining the basophil activation for each of the individual aliquots i) to v) from step b),
d) determining the change of CD63 expression and CD203c expression in the individual aliquots i)-iv) compared to the control v); and
e) calculating a combined symptom and tolerance class (CSTC) as a measure for the prevalence and severity of an allergy against the allergy-triggering source for the individual based on the data determined in steps c) and d).

2. The method according to claim 1 for differentiating between allergic individuals and sensitized but non-allergic individuals.

3. The method according to any one of the preceding claims, wherein the allergy-triggering source is selected from the group of one or more food, inhalation or environmental allergy triggering source, preferably, wherein the allergy-triggering source is selected from peanut, dust mite, soy, milk, egg, fish, mussels, meat, tree nuts, wheat, apple, bee, wasp, cat, dog, weeds, grasses and trees.

4. The method according to any one of the preceding claims, like wherein the allergy-triggering source is peanut, the extract is an alkaline peanut extract; the major allergen is Ara h 2, the optional minor allergen is Ara h 8, and the lipid-associated allergens are oleosins containing at least Ara h 14 and Ara h 15 from roasted peanuts.

5. The method according to any one of the preceding claims wherein the sample is selected from fluid or enzymatically digested tissue for single cell suspensions of said subject, in particular, the sample is selected from sputum, blood including whole blood, saliva, nasal secretion and urine, preferably the sample is whole blood.

6. The method according to any one of the preceding claims wherein the method for determining activation of basophils is a cytometric-based *in vitro* method using the fluorescence-labelled markers anti-CD63, anti- FcεR1a, and anti-CD203c, preferably, wherein the change of CD63 expression and/or CD203c expression is determined as a change of median fluorescence intensity in cytometric measurement.

7. The method according to claim 5 or 6 wherein the basophil activation is determined as proportion of activated basophils using the identification marker CD63, FcεR1a, and CD203c along with the following gating strategy:
i) expression of FcεR1a and CD203c;
ii) forward scatter area (FSC-A) vs. sideward scatter area (SSC-A);
iii) sideward scatter width (SSC-W) vs. CD203c;
and the proportion of activated basophils is calculated based on CD63-, FcεR1a- and CD203c-positive cells.

8. The method according to any one of the preceding claims further comprising a step of excluding individuals not accessible to CSTC classification wherein said individuals are **characterized by** non-responsiveness to IgE mediated basophil activation, in particular, determined by incubation of a single aliquot with a single IgE-independent activation stimulus, like fMLP, and/or incubation of a single aliquot with a single IgE-dependent activation stimulus, like a mixture of two anti-IgE antibodies.

9. The method according to any one of the preceding claims wherein at least part of the steps is conducted by a computer-implemented analysis method.

10. The method according to any of the preceding claims wherein different stages of CSTC are calculated based on the degree of basophil activation obtained for the individual stimuli defined under claim 1, step b) i)-iv)), as well as predetermined thresholds for the change in CD63 expression and CD203c expression, preferably, where the CTSC classifies in ascending order the severity of an individual's allergy from 0 to 3, and the stages being determined as follows:
i) CSTC 0 with reactivity only to extract i) with a proportion of activated basophils less than 25% indicating no allergy with high tolerance to the allergy-triggering source;
ii) CSTC 1 with reactivity to not more than three stimuli i) to iv) with individual proportions of activated basophils less than 25%, respectively, and the change in CD63 expression and/or CD203c expression, in particular, a delta MFI, for the stimuli is less than 2-fold, indicating a mild allergy with substantial tolerance;
iii) CSTC 2 with reactivity to not more than three stimuli i) to iv) with individual proportions of activated basophils only for one stimulus exceeding 30% and the change in CD63 expression and/or CD203c expression, in particular, a delta MFI, for most stimuli is less than 2.5-fold, indicating a moderate allergy with moderate tolerance; and
iv) CSTC 3 with reactivity to at least three stimuli i) to iv) with individual proportions of activated basophils of at least two allergens exceeding 30% and the change in CD63 expression and/or CD203c expression, e.g., a delta MFI for most stimuli above 2.5-fold, indicating a severe allergy with low or no tolerance.

11. A computer-implemented method for determining the prevalence and/or severity of an allergy of an individual suspected to be afflicted with allergy against an allergy-triggering source comprising the step of
a) obtaining data of measured parameters, preferably by flowcytometry, to determine the percentage or absolute number of activated basophils at least in individual samples i) to v) as defined in claim 1 of the individual, wherein the basophil activation is determined using the identification marker CD63, FcεR1a, and CD203c;
b) computing the data of step a);
c) identifying the percentage or absolute number of activated CD63 expressing basophils, the change of CD63 expression, and the change of CD203c expression of said activated basophils compared to the control v) as defined in claim 1;
d) determining the allergy status and/or severity, optionally showing the data on an output unit.

12. The method according to any one of the preceding claims wherein the sample processing and measurement are conducted automatically using a flow cytometer with integrated robotic sample handling and manipulation, and wherein the basophil activation is optionally determined using a computer-implemental method according to any one of claims 9 to 11.

13. Computer-readable medium or computer program product having computerexecutable instructions for performing the steps as identified in any one of claims 9 to 11.

14. The use of a test kit or kit of parts for determining the prevalence and/or severity of an allergy in an induvial suspected to be afflicted with allergy against an allergy-triggering source comprising anti-CD-203c, anti-FcεRIα and anti-CD63 antibodies, each labelled with a distinct fluorochrome, optionally lysis buffer for erythrocytes and, optionally, washing buffer, and, in addition instruction on how to use the test or kit of parts in a method according to any one of claims 1 to 9, preferably further comprising at least the extract of the allergen triggering source, the major allergen, and one lipid-associated allergen being different to the major allergen.

15. A method according to any of the preceding claims wherein the CSTC determined is used to guide behavioral recommendations and/or therapy decisions for the individual.
